# EUROPEAN PATENT APPLICATION

(11) **EP 3 165 170 A1**
(43) Date of publication of application: **10.05.2017**
(21) Application number: 15865845.0
(22) Date of filing: 02.11.2015
(51) Int. Cl.: A61B 8/12

(54) **ULTRASONIC ENDOSCOPE**

(30) Priority: 01.12.2014 JP 2014243427
(71) Applicant: Olympus Corporation, Hachioji-shi Tokyo 192-8507 (JP)
(72) Inventor: KITAHARA Toshihiro, Tokyo 192-8507 (JP)
(74) Representative: Gunzelmann, Rainer
(86) International application number: PCT/JP2015/080897
(87) International publication number: WO 2016/088496

(57) **Abstract**

An ultrasound endoscope includes: an insertion portion provided with a housing portion 34 on a distal end side; an ultrasound transmitting/receiving portion provided in the housing portion 34 and configured to transmit and receive ultrasound to and from the observation site; a cable 41 configured to transmit electric signals inputted to and outputted from the ultrasound transmitting/receiving portion; an inserting portion 36 configured to cause the cable 41 to extend from an inside of the housing portion 34; and a fitted member 37 fitted on an outer face 36g of an outer peripheral side potion of the inserting portion 36 so as to cover at least a part of the outer face 36g along an extension direction E.

## Description

### Technical Field

The present invention relates to an ultrasound endoscope with a cable extended from an ultrasound transmitting/receiving portion positioned inside a housing portion provided on a distal end side of an insertion portion to an outside of the housing portion via an inserting portion.

### Background Art

An ultrasound endoscope configured to obtain an ultrasound image by transmitting/receiving ultrasound to and from an observation site is provided with an ultrasound probe.

The ultrasound probe is provided with an ultrasound transmitting/receiving portion positioned in an insulative housing portion provided on a distal end side in an extension direction (hereinafter simply referred to as the distal end side) of an insertion portion of an ultrasound endoscope and configured to transmit/receive ultrasound; and a cable extended from the ultrasound transmitting/receiving portion.

The housing portion internally retains the ultrasound transmitting/receiving portion such that an ultrasound transmitting/receiving surface of the ultrasound transmitting/receiving portion is exposed outside the housing portion.

Further, as for the cable, an outer periphery of the cable is covered with an insulating tube or the like in order to ensure electrical safety of the cable. The cable is inserted through the insertion portion, an operation portion, a universal cord and an endoscope connector of the ultrasound endoscope, and a connector provided on an extension end side of the cable is electrically connected to a substrate for connection with an ultrasound observation apparatus in the endoscope connector.

Here, Japanese Patent Application Laid-Open Publication No. 2014-18282 discloses a configuration in which an inserting portion cylindrically extended rearward in an extension direction (hereinafter simply referred to as rearward) of the housing portion from the housing portion is provided.

A distal end side of the insulating tube is fixed to the inserting portion, and the inserting portion is configured to, by causing a cable to extend from an inside of the housing portion and covering a distal end side of the cable outside the housing portion, prevent the distal end side of the cable inserted inside from being bent.

Here, at time of assembling the ultrasound probe, work of causing the cable to be inserted into the inserting portion from the housing portion side, that is, from frontward in the extension direction (hereinafter simply referred to as frontward) to rearward is performed, and there is a problem that, if the number of cables is large, and an inner diameter of the inserting portion is small, workability is poor.

Note that such a problem can be solved by increasing the inner diameter of the inserting portion, because a clearance between the cables and an inner face of an outer peripheral side portion of the inserting portion is increased.

However, when the diameter of the inserting portion is increased, there is a problem that a diameter of the distal end side of the insertion portion on which the inserting portion is provided is also increased, accompanying the increase in the diameter of the inserting portion, because various members are provided highly densely in the distal end side of the insertion portion of the ultrasound endoscope.

Note that, since the insertion portion of the ultrasound endoscope is inserted into an observation site, reduction in the diameter of the distal end side of the insertion portion has been pursued in 0.1 mm increments. Therefore, increase in the diameter of the distal end side of the insertion portion is not preferable.

Further, though it is possible to increase the clearance between the cable and the inner face of the outer peripheral side portion of the inserting portion by thinning a thickness of the outer peripheral side portion of the inserting portion, it is not possible to thin the outer peripheral side portion of the inserting portion to be of a prescribed thickness or thinner because a minimum thickness of a member covering a cable is prescribed from a viewpoint of electrical safety standards for ensuring voltage resistance of the cable.

Furthermore, in order to realize higher image quality of the ultrasound endoscope, the number of ultrasound transducers constituting the ultrasound transmitting/receiving portion can be increased. However, when the number of ultrasound transducers increases, the number of cables electrically connected to the ultrasound transducers also increases, and, therefore, the clearance between the cables and the inner face of the outer peripheral side portion of the inserting portion becomes smaller, and there is a problem that the workability at the time of causing the cables to be inserted into the inserting portion becomes poorer.

The present invention has been made in view of the problems and the situation described above, and an object is to provide an ultrasound endoscope provided with a configuration capable of ensuring the electrical safety standards of cables in the inserting portion without damaging the workability at the time of causing the cables to be inserted into the inserting portion, and realizing reduction in the diameter of the distal end side of the insertion portion.

### Disclosure of Invention

### Means for Solving the Problem

An ultrasound probe according to an aspect of the present invention includes: an insertion portion configured to be inserted into an observation site and provided with a housing portion on a distal end side in an extension direction; an ultrasound transmitting/receiving portion provided in the housing portion and configured to transmit and receive ultrasound to and from the observation site; a cable extended rearward in the extension direction from the ultrasound transmitting/receiving portion and configured to transmit electric signals to be inputted to and outputted from the ultrasound transmitting/receiving portion; an inserting portion extended rearward in the extension direction from the housing portion, having a substantially cylindrical shape, with a slit along the extension direction formed on an outer peripheral side potion, and configured to cause the cable to extend from an inside of the housing portion; and a fitted member fitted on an outer face of the outer peripheral side potion of the inserting portion so as to cover at least a part of the outer face along the extension direction.

### Brief Description of the Drawings

Fig. 1 is a diagram showing an ultrasound endoscope apparatus provided with an ultrasound endoscope of a first embodiment;
Fig. 2 is a diagram showing an ultrasound probe of Fig. 1;
Fig. 3 is a partial perspective view showing a distal end side of the ultrasound probe of Fig. 2 from which an insulating tube has been removed;
Fig. 4 is a partial perspective view showing the distal end side of the ultrasound probe of Fig. 3 from which a fitted member has been removed;
Fig. 5 is a perspective view showing only a housing portion and an inserting portion extracted from the distal end side of the ultrasound probe of Fig. 3;
Fig. 6 is a partial cross-sectional view of the distal end side of the ultrasound probe along a VI-VI line in Fig. 3;
Fig. 7 is a partial cross-sectional view showing a state before the fitted member is covered over an outer face of an outer peripheral side portion of the inserting portion on the distal end side of the ultrasound probe of Fig. 6;
Fig. 8 is a partial perspective view showing a modification of the distal end side of the ultrasound probe in which the fitted member of Fig. 3 has a shape shorter than shown in Fig. 3;
Fig. 9 is a perspective view showing a modification of a slit formation position on the housing portion and the inserting portion of Fig. 5;
Fig. 10 is a perspective view showing a modification of the number of slits formed on the housing portion and the inserting portion of Fig. 5;
Fig. 11 is a partial perspective view showing a modification of the distal end side of the ultrasound probe in which the fitted member of Fig. 3 is configured with a distal end rigid member;
Fig. 12 is a partial perspective view showing the distal end side of the ultrasound probe of an ultrasound endoscope of a second embodiment from which the insulating tube has been removed;
Fig. 13 is a diagram of the distal end side of the ultrasound probe of Fig. 12 when seen from a XIII direction in Fig. 12;
Fig. 14 is a partial perspective view showing the distal end side of the ultrasound probe of Fig. 12 from which the fitted member has been removed;
Fig. 15 is a diagram of the distal end side of the ultrasound probe of Fig. 14 when seen from a XV direction in Fig. 14;
Fig. 16 is a perspective view showing only the housing portion and the inserting portion extracted from the distal end side of the ultrasound probe of Fig. 12;
Fig. 17 is a partial perspective view showing the distal end side of the ultrasound probe of an ultrasound endoscope of a third embodiment from which the insulating tube has been removed;
Fig. 18 is a partial perspective view showing a state in which the fitted member is pulled out from the outer face of the outer peripheral side portion of the inserting portion on the distal end side of the ultrasound probe of Fig. 17;
Fig. 19 is a partial perspective view showing the distal end side of the ultrasound probe of an ultrasound endoscope of a fourth embodiment by extracting only the housing portion and the inserting portion;
Fig. 20 is a partial perspective view showing a state in which a watertight member is fitted in a groove of the inserting portion of Fig. 19;
Fig. 21 is a partial cross-sectional view of the distal end side of the ultrasound probe when the distal end rigid member is fitted on the outer face of the outer peripheral side portion of the inserting portion of Fig. 20;
Fig. 22 is a partial perspective view showing the distal end side of the ultrasound probe of an ultrasound endoscope of a fifth embodiment from which the insulating tube has been removed;
Fig. 23 is a partial perspective view showing the distal end side of the ultrasound probe of Fig. 22 from which the fitted member has been removed;
Fig. 24 is a partial perspective view showing only the housing portion and the inserting portion extracted from the distal end side of the ultrasound probe of Fig. 22;
Fig. 25 is a partial perspective view showing a state in which cables and connectors are caused to be close to the housing portion and the inserting portion of Fig. 9 from frontward;
Fig. 26 is a partial perspective view showing a state in which the cables and the connectors of Fig. 25 are caused to be inserted into the housing portion and the inserting portion; and
Fig. 27 is a diagram showing sections of the housing portion and the inserting portion along a IIXVII-IXVII line in Fig. 26 together with the cables and the connectors.

### Best Mode for Carrying Out the Invention

Embodiments of the present invention will be described below with reference to drawings. Note that the drawings are schematic, and it should be noticed that a relationship between thickness and width of each member, a thickness ratio among respective members and the like are different from actual ones; and, among the mutual drawings, portions having a different dimensional relationship or ratio are, of course, included.

### (First embodiment)

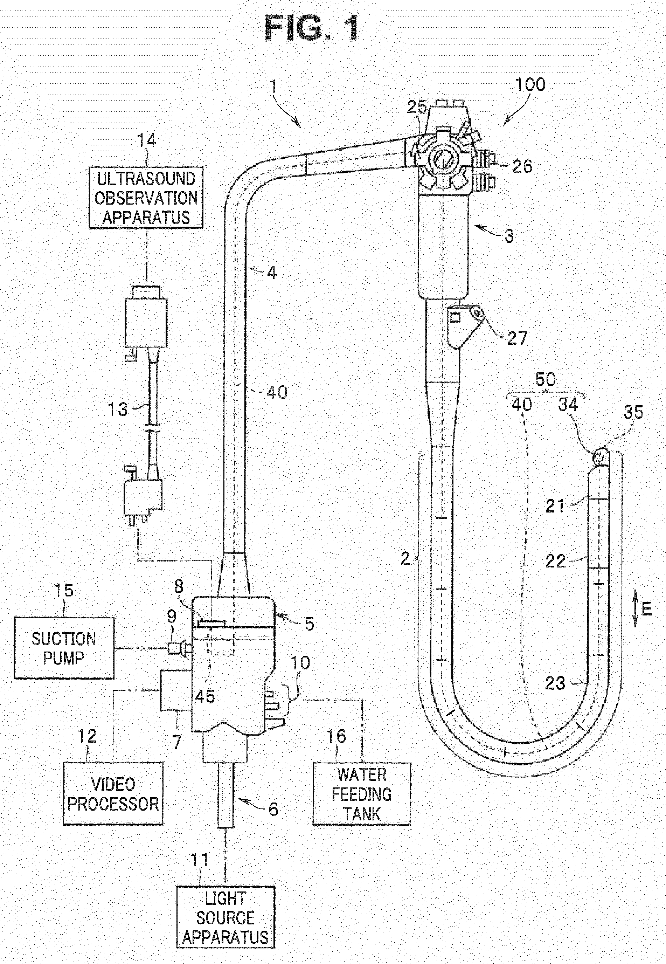

Fig. 1 is a diagram showing an ultrasound endoscope apparatus provided with an ultrasound endoscope of the present embodiment.

As shown in Fig. 1, a main part of an ultrasound endoscope apparatus 100 is configured with an ultrasound endoscope 1, a light source apparatus 11, a video processor 12, an ultrasound observation apparatus 14, a suction pump 15 and a water feeding tank 16.

A main part of the ultrasound endoscope 1 is configured with an elongated insertion portion 2 configured to be inserted into an observation site and provided with a housing portion 34 on a distal end side; an operation portion 3 provided on a proximal end in an extension direction E (hereinafter simply referred to as the proximal end) of the insertion portion 2; a universal cord 4 extended from the operation portion 3, and an endoscope connector 5 provided on an extension end of the universal cord 4.

The endoscope connector 5 is provided with a light source connector 6, an electric connector 7, an ultrasound connector 8, a suction pipe sleeve 9 and an air/water feeding pipe sleeve 10.

A configuration is adopted in which the light source apparatus 11 configured to supply illuminating light is attachable to and detachable from the light source connector 6. Further, a configuration is adopted in which the video processor 12 configured to perform various signal processing and the like via a signal cable not shown is attachable to and detachable from the electric connector 7.

A configuration is adopted in which the ultrasound observation apparatus 14 is attachable to and detachable from the ultrasound connector 8 via an ultrasound cable 13.

Further, a configuration is adopted in which the suction pump 15 is attachable to and detachable from the suction pipe sleeve 9 via a suction tube not shown. Furthermore, a configuration is adopted in which the water feeding tank 16 is attachable to and detachable from the air/water feeding pipe sleeve 10 via an air/water feeding tube not shown.

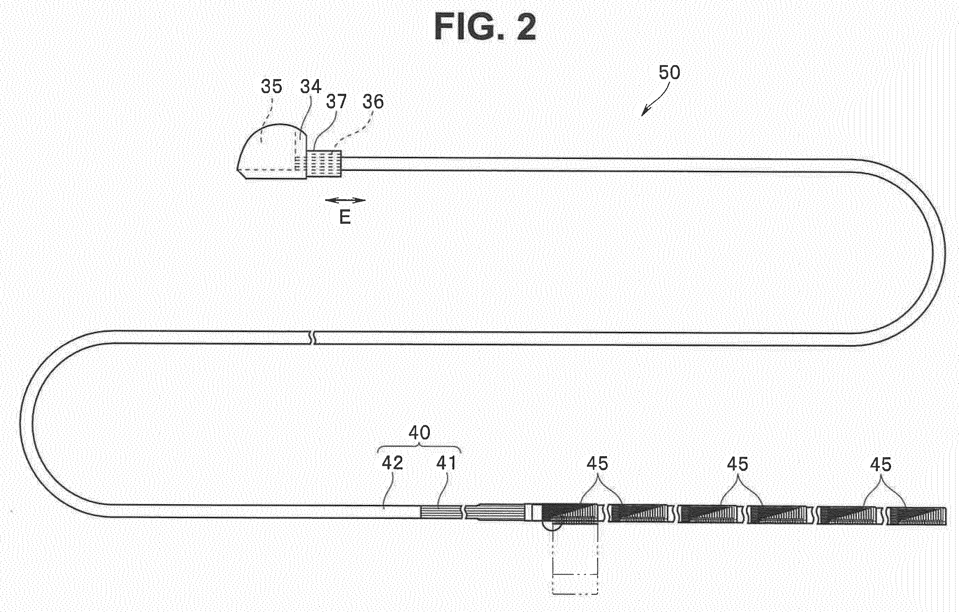

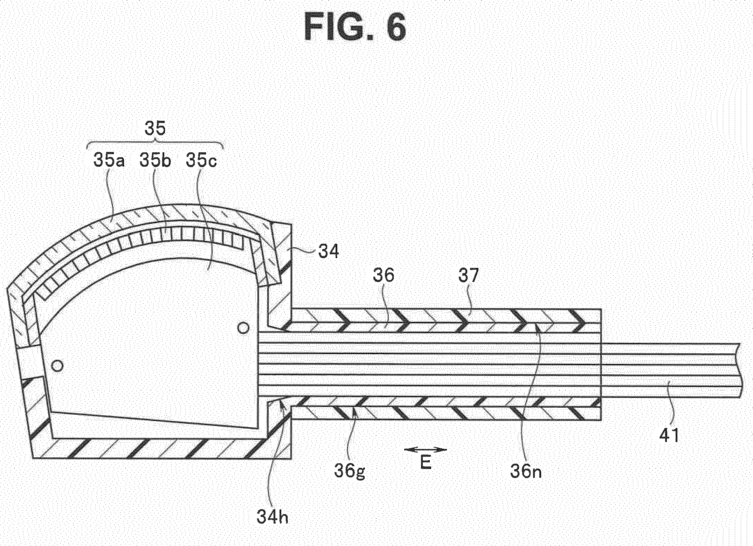

The ultrasound observation apparatus 14 is such that transmits electric signals to an ultrasound transmitting/receiving portion 35 to be described later via the ultrasound cable 13 and cables 41 to be described later (see Fig. 2) and such that electric signals are inputted from the ultrasound transmitting/receiving portion 35 via the ultrasound cable 13 and the cables 41. For example, the ultrasound observation apparatus 14 performs an operation of performing drive control of ultrasound transducers 35b to be described later (see Fig. 6) and signal processing of an electric signal acquired by the drive control of the ultrasound transducers 35b to generate a video signal.

Note that the video signal generated by the ultrasound observation apparatus 14 is outputted to a display apparatus not shown. As a result, an ultrasound image is displayed on a screen of the display apparatus which has received the video signal.

The insertion portion 2 of the ultrasound endoscope 1 is configured with a distal end portion 21 internally provided with an image pickup unit, an illumination unit and the like not shown, a bending portion 22 configured to be bendable, for example, in upward and downward directions and left and right directions, and a long flexible tube portion 23 having flexibility, which are connectedly arranged in that order from the distal end side. Note that at least a part of the housing portion 34 is exposed on a distal end side of the distal end portion 21.

The operation portion 3 is provided with bending operation knobs 25, 26 for performing a bending operation of the bending portion 22. Further, at a position on the insertion portion 2 side of the operation portion 3, a treatment instrument insertion opening 27 is provided which is for introducing a treatment instrument into a body via a treatment instrument insertion conduit provided in the insertion portion 2 and the operation portion 3, which is not shown.

The video processor 12 is such that generates a standard video signal by performing signal processing for an electric signal transmitted from the image pickup unit provided in the distal end portion 21, which is not shown, and outputs the video signal to the display apparatus not shown to cause an endoscopic observation image to be displayed on the screen of the display apparatus.

Further, the ultrasound endoscope 1 is provided with an ultrasound probe 50 having the housing portion 34.

Next, a configuration of the ultrasound probe 50 will be described with reference to Fig. 2. Fig. 2 is a diagram showing the ultrasound probe of Fig. 1.

As shown in Fig. 2, the ultrasound probe 50 is provided with the housing portion 34, the ultrasound transmitting/receiving portion 35 provided in the housing portion 34, an inserting portion 36, a fitted member 37 and an ultrasound transducer cable 40.

A main part of the ultrasound transducer cable 40 is configured with the cables 41 configured with a plurality of signal lines and configured to transmit electric signals inputted to and outputted from the ultrasound transmitting/receiving portion 35, and an insulating tube 42 configured to cover an outer periphery of the cables 41. The cables 41 configured with the plurality of signal lines are bundled as one ultrasound transducer cable 40 by the insulating tube 42.

Note that, in order to ensure prescribed voltage resistance of the cables 41 and prevent flexibility of the insertion portion 2 from being hindered when the ultrasound probe 50 is provided on the ultrasound endoscope 1, the insulating tube 42 is configured, for example, with a heat-shrinkable tube formed with rubber or the like which has voltage resistance and which is thin and flexible.

Furthermore, the insulating tube 42 has a function of preventing the cables 41 from coming into contact with metal members such as a light guide, a balloon conduit, a forceps channel and the like provided in the insertion portion 2, which are not shown, when the ultrasound probe 50 is provided on the ultrasound endoscope 1. That is, the insulating tube 42 has a function of insulating the cables 41 against other metal members.

Further, on a proximal end side in the extension direction E (hereinafter simply referred to as the proximal end side) of the ultrasound transducer cable 40, the cables 41 are exposed because the outer periphery of the cables 41 is not covered with the insulating tube 42, and a plurality of connectors 45 configured with flexible substrates or the like for electrically connecting the cables 41 to a substrate of the ultrasound connector 8 provided in the endoscope connector 5, which is not shown, are provided on the exposed part of the cables 41.

Distal ends in the extension direction E (hereinafter simply referred to as the distal end) of the cables 41 are introduced into an inside of the housing portion 34 via the inserting portion 36 and electrically connected to the ultrasound transmitting/receiving portion 35 in the housing portion 34. That is, the cables 41 are extended from the ultrasound transmitting/receiving portion 35 in the housing portion 34 to rearward via the inserting portion 36.

Note that a distal end side of the insulating tube 42 may be positioned, covering the outer periphery of the fitted member 37 or may be positioned at a same position as a proximal end of the inserting portion 36 without covering the outer peripheries of the inserting portion 36 and the fitted member 37.

In this case, however, it is necessary for the insulating tube 42 to cover the cables 41 without a gap relative to the cables 41, that is, without the cables 41 being exposed in order to ensure the prescribed voltage resistance of the cables 41.

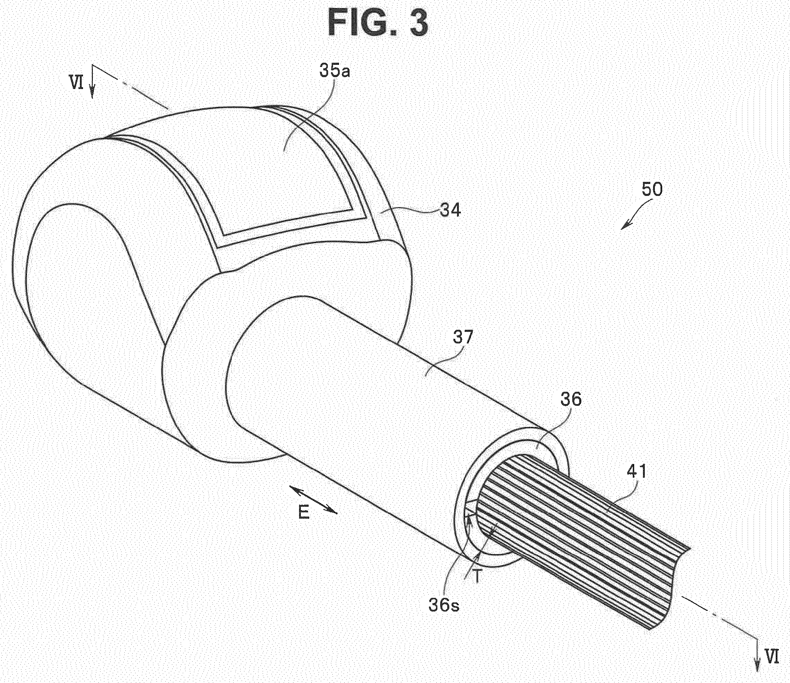

Next, a configuration of a distal end side of the ultrasound probe 50 will be described with use of Figs. 3 to 7 together with Fig. 2.

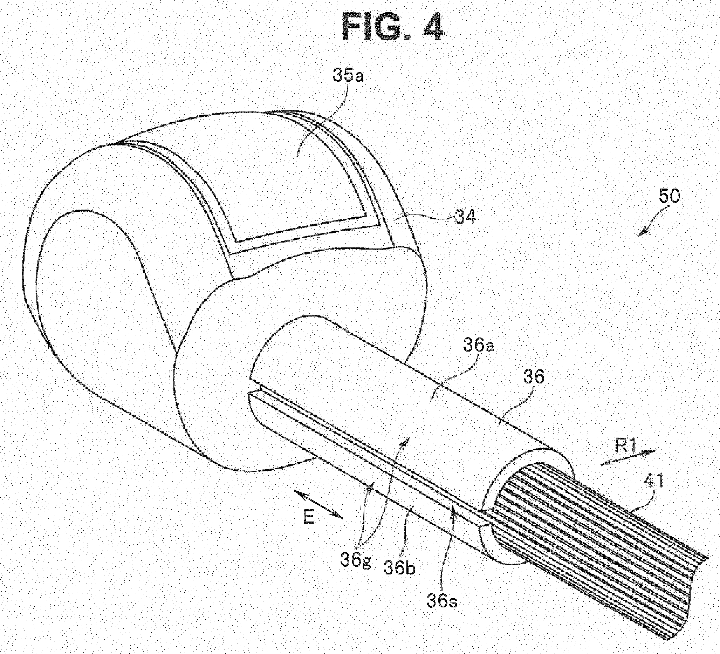

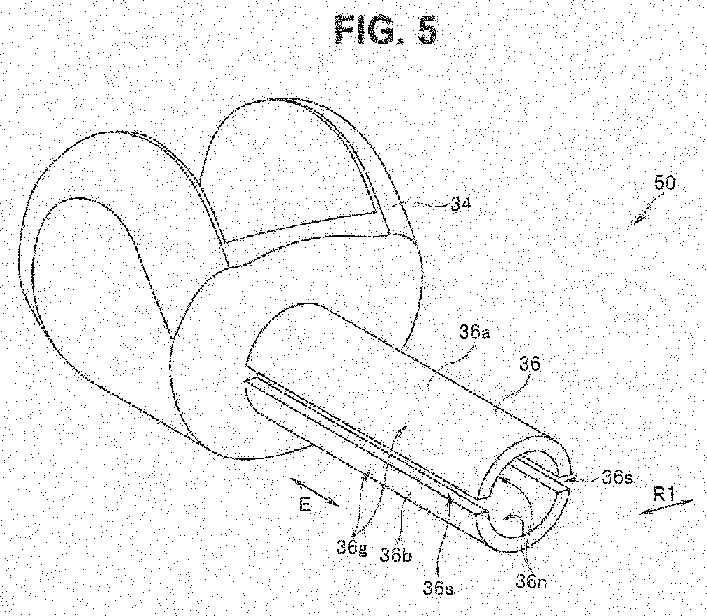

Fig. 3 is a partial perspective view showing the distal end side of the ultrasound probe of Fig. 2 from which the insulating tube has been removed; Fig. 4 is a partial perspective view showing the distal end side of the ultrasound probe of Fig. 3 from which the fitted member has been removed; and Fig. 5 is a perspective view showing only the housing portion and the inserting portion extracted from the distal end side of the ultrasound probe of Fig. 3.

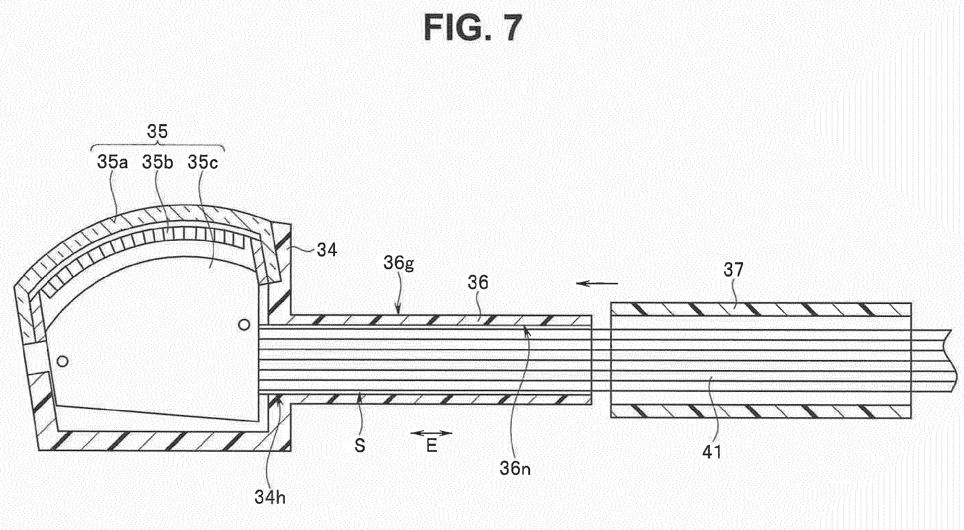

Further, Fig. 6 is a partial cross-sectional view of the distal end side of the ultrasound probe along a VI-VI line in Fig. 3; and Fig. 7 is a partial cross-sectional view showing a state before the fitted member is covered over the outer face of the outer peripheral side portion of the inserting portion on the distal end side of the ultrasound probe of Fig. 6.

As shown in Figs. 2, 6 and 7, the ultrasound transmitting/receiving portion 35 configured to transmit and receive ultrasound to and from an observation site is provided inside the housing portion 34. Note that, as shown in Figs. 6 and 7, the housing portion 34 retains the ultrasound transmitting/receiving portion 35 such that an acoustic lens 35a to be an ultrasound transmitting/receiving surface of the ultrasound transmitting/receiving portion 35, which is to be described later, is exposed.

Further, the housing portion 34 is configured with a member having voltage resistance, for example, a member made of resin, for example, polysulphone, polyimide or PET in order to ensure the prescribed voltage resistance of the cables 41 similarly to the insulating tube 42.

As shown in Figs. 6 and 7, the ultrasound transmitting/receiving portion 35 is provided with the acoustic lens 35a, the ultrasound transducers 35b and a substrate 35c.

The ultrasound transducers 35b are such that radiate ultrasound to an observation site via the acoustic lens 35a and that receives the ultrasound reflected from the observation site after radiation, via the acoustic lens 35a and convert the ultrasound to an electric signal.

The acoustic lens 35a is such that has a function of collecting the ultrasound radiated from the ultrasound transducers 35b to the observation site so that the ultrasound does not diffuse and radiates the ultrasound.

The substrate 35c is electrically connected to the ultrasound transducers 35b, and the distal ends of the cables 41 which are not covered with the insulating tube 42, that is, which are exposed are electrically connected to the substrate 35c on a distal end side of the ultrasound transducer cable 40.

Thereby, the electric signal converted from the ultrasound by the ultrasound transducers 35b is transmitted to the cables 41 via the substrate 35c.

Further, as shown in Figs. 6 and 7, on a proximal end side of the housing portion 34, a hole portion 34h for guiding the cables 41 the distal ends of which are electrically connected to the substrate 35c from the inside of the housing portion 34 to an outside of the housing portion 34.

Here, as shown in Figs. 2 to 7, the inserting portion 36 communicating with the hole portion 34h and extended rearward, having a cylindrical shape is provided on a proximal end face of the housing portion 34.

Note that, though Figs. 6 and 7 show a case where the inserting portion 36 is formed integrally with the housing portion 34 as an example, the inserting portion 36 may be a member separated from the housing portion 34 fixed to the proximal end face of the housing portion 34.

The inserting portion 36 is such that causes the cables 41 to be extended from the housing portion 34. The inserting portion 36 is inserted in the distal end portion 21 and is configured with a member having voltage resistance, for example, a member made of resin, for example, polysulphone, polyimide or PET in order to ensure the prescribed voltage resistance of the cables 41 similarly to the housing portion 34b. As shown in Fig. 3, the inserting portion 36 has a minimum thickness T to ensure the electrical safety standards of the cables 41 and has a function of preventing other metal members from coming into contact with the cables 41.

Note that, as the thickness T to ensure the electrical safety standards, for example, a thickness of about 0.4 to 0.5 mm is given as an example.

Further, as shown in Figs. 4 and 5, for example, two slits 36s along the extension direction E are formed on the outer peripheral side portion of the inserting portion 36 in a direction orthogonal to a direction of an array of the ultrasound transducers 35b (hereinafter referred to as a lateral direction) R1 from a distal end to proximal end of the inserting portion 36, facing each other. Note that the number of slits 36s may be one.

Further, by the two slits 36s being formed on the outer peripheral side portion of the inserting portion 36, the outer peripheral side portion of the inserting portion 36 is separated into two partial arc-shaped small pieces 36a, 36b. The small pieces 36a, 36b can be elastic to an inner side and an outer side in a diameter direction by the two slits 36s.

As shown in Figs. 3, 6 and 7, the cylindrical-shaped fitted member 37 inserted in the distal end portion 21 is covered and fitted on each of outer faces 36g of the small pieces 36a, 36b of the inserting portion 36 along the extension direction E from the distal end to proximal end of the inserting portion 36.

The fitted member 37 is such that, by causing the small pieces 36a, 36b to be elastically deformed to the inner side in the diameter direction via the slits 36s as shown in Fig. 6, eliminates or minimizes a clearance S which has occurred between the outer periphery of the cables 41 and each of inner faces 36n of the small pieces 36a, 36b as shown in Fig. 6.

Further, the fitted member 37 is configured with a member having voltage resistance, for example, a member made of resin, for example, polysulphone, polyimide or PET in order to ensure the prescribed voltage resistance of the cables 41 similarly to the inserting portion 36 and is formed thinner than the inserting portion 36 and the clearance S, for example, formed to be of a thickness of about 0.1 mm.

Therefore, the fitted member 37 may be caused to function as a member for ensuring the prescribed voltage resistance of the cables 41 together with the inserting portion 36.

Note that, since other components of the ultrasound probe 50 are same as those of an already-known ultrasound probe, description of the components will be omitted.

Next, operation of the present embodiment will be described.

At time of assembling the ultrasound probe 50, first, a worker causes a proximal end side of the cables 41 to be inserted into the housing portion 34 and the inserting portion 36 shown in Fig. 5 from frontward of the housing portion 34, and fixes the acoustic lens 35a to the housing portion 34 after the distal ends of the cables 41 are caused to be inserted.

In the work, at time of causing the cables 41 to pass through the inserting portion 36, it is difficult to perform the work if the number of the cables 41 is large, and the clearance S between the outer periphery of the cables 41 and the inner face 36n of the outer peripheral side portion of the inserting portion 36 is small as described above.

In the present embodiment, however, since the slits 36s are formed on the outer peripheral side portion of the inserting portion 36, the worker can increase the clearance S by causing the small pieces 36a, 36b to be elastically deformed and spread to the outer side in the diameter direction via the slits 36s, and, therefore, insertability of the cables 41 into the inserting portion 36 is improved.

After fixing the acoustic lens 35a to the housing portion 34, the worker covers the fitted member 37 over the outer faces 36g of the small pieces 36a, 36b from rearward as shown in Figs. 6 and 7.

As a result, by the small pieces 36a, 36b being elastically deformed to the inner side in the diameter direction via the slits 36s, the clearance S which has occurred between the outer periphery of the cables 41 and each of the inner faces 36n of the small pieces 36a, 36b is eliminated or minimized, and, therefore, a diameter of the inserting portion 36 is reduced as shown in Fig. 6.

Note that, since other assembly works for the ultrasound probe 50 are well known, description of the assembly works will be omitted.

Thus, in the present embodiment, it has been shown that the slits 36s are formed along the extension direction E from the distal end to proximal end of the inserting portion 36, on the outer peripheral side portion of the inserting portion 36. Further, it has been shown that the fitted member 37 is fitted on the outer face 36g of the outer peripheral side potion of the inserting portion 36.

According to the above, at the time of performing the work of inserting the cables 41 into the inserting portion 36, the worker can increase the clearance S between the outer periphery of the cables 41 and the inner face 36n of the inserting portion 36 even if the number of the cables 41 increases, by causing the small pieces 36a, 36b of the outer peripheral side portion of the inserting portion 36 formed by the slits 36s to be elastically deformed to the outer side in the diameter direction, and, therefore, the insertability of the cables 41 into the inserting portion 36 is improved.

Further, after the cables 41 are inserted into the inserting portion 36, the small pieces 36a, 36b are elastically deformed to the inner side in the diameter direction via the two slits 36s by the fitted member 37 being fitted over the outer face 36g of the outer peripheral side portion of the inserting portion 36, and, therefore, the clearance S between the outer periphery of the cables 41 and the inner face 36n of the inserting portion 36 is eliminated or minimized.

Therefore, because the clearance S can be substantially ignored, it does not happen that the diameter of the inserting portion 36 becomes larger than the minimum thickness of the outer peripheral side portion to ensure the electrical safety standards of the cables 41. Simultaneously, since the fitted member 37 is thinner than the clearance S, reduction in a diameter of the distal end portion 21 provided with the inserting portion 36 can be realized.

Furthermore, since the clearance S is substantially eliminated, it is possible to prevent positional displacement of the cables 41 in the extension direction E inside the inserting portion 36 by the inner face 36n of the outer peripheral side potion of the inserting portion 36 which is in contact with the outer periphery of the cables 41.

From the above, it is possible to provide the ultrasound endoscope 1 provided with the configuration capable of ensuring the electrical safety standards of the cables 41 in the inserting portion 36 without damaging the workability at the time of causing the cables 41 to be inserted into the inserting portion 36, and realizing reduction in the diameter of the distal end side of the insertion portion 2.

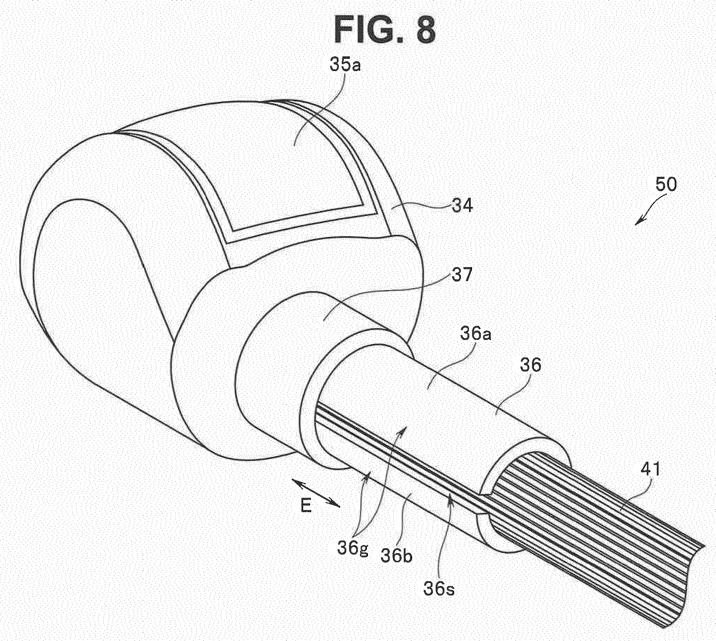

Note that a modification will be shown below with use of Fig. 8. Fig. 8 is a partial perspective view showing a modification of the distal end side of the ultrasound probe in which the fitted member of Fig. 3 has a shape shorter than shown in Fig. 3.

In the present embodiment described above, it has been shown that the fitted member 37 is covered and fitted over the outer face 36g of the outer peripheral side potion of the inserting portion 36 in the extension direction E from the distal end to the proximal end.

This is not limiting. The fitted member 37 may have a shape shorter than shown in Fig. 3, being fitted only on a part of the outer face 36g of outer peripheral side potion of the inserting portion 36, which is in contact with the housing portion 34, as shown in Fig. 8 if it is possible to cause the small pieces 36a, 36b of the inserting portion 36 to be elastically deformed to the inner side in the diameter direction.

That is, the fitted member 37 is only required to be fitted so as to be covered over at least a part of the outer face of the outer peripheral side potion of the inserting portion 36 along the extension direction E.

Effects similar to those of the present embodiment can be also obtained by such a configuration.

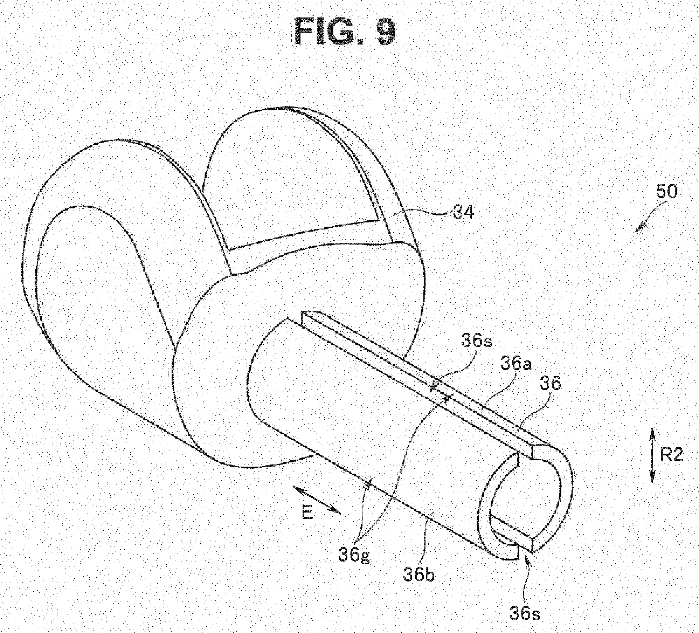

Further, another modification will be shown below with use of Fig. 9. Fig. 9 is a perspective view showing a modification of a slit formation position on the housing portion and the inserting portion of Fig. 5.

In the present embodiment described above, it has been shown that the two slits 36s are formed on the outer peripheral side potion of the inserting portion 36 facing each other in a lateral direction R1 to form the small pieces 36a, 36b.

This is not limiting. As shown in Fig. 9, the two slits 36s may be formed on the outer peripheral side potion of the inserting portion 36 facing each other in an array direction (hereinafter referred to as the longitudinal direction) R2 of the ultrasound transducers 35b to form the small pieces 36a, 36b.

Thus, even if the two slits 36s are formed in the longitudinal direction R2, effects similar to those of the present embodiment described above can be obtained.

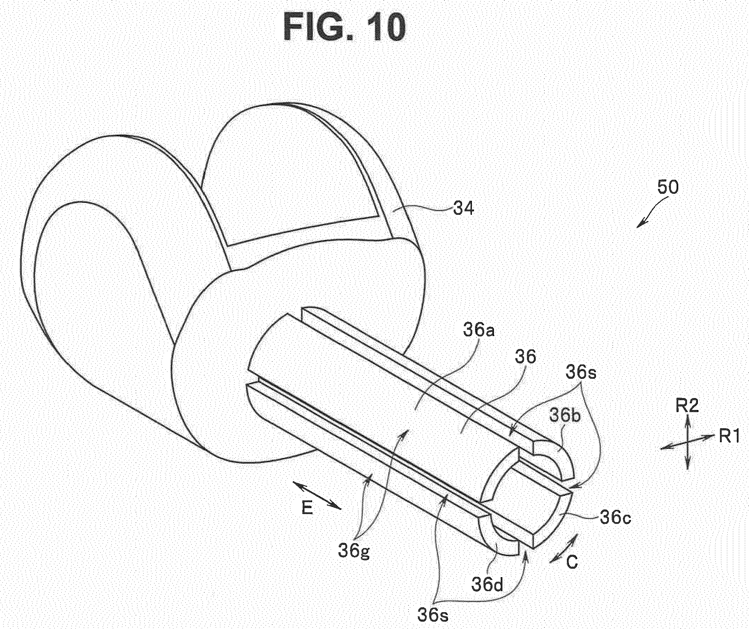

Furthermore, another modification will be shown below with use of Fig. 10. Fig. 10 is a perspective view showing a modification of the number of slits formed on the housing portion and the inserting portion of Fig. 5.

In the present embodiment described above, it has been shown that the two slits 36s are formed on the outer peripheral side potion of the inserting portion 36 facing each other in the lateral direction R1 to form the two small pieces 36a, 36b.

Further, in Fig. 9, it has been shown that the two slits 36s are formed on the outer peripheral side potion of the inserting portion 36 facing each other in the longitudinal direction R2 to form the two small pieces 36a, 36b.

This is not limiting. As shown in Fig. 10, it is also possible to form two slits 36s on the outer peripheral side potion of the inserting portion 36 facing each other in the lateral direction R1, and two slits 36s facing each other in the longitudinal direction R2, that is, form the slits 36s in a cross shape at equal intervals in a circumferential direction C so that four partial arc-shaped small pieces 36a, 36b, 36c, 36d may be formed.

Thus, even if the four slits 36s are formed, effects similar to those of the present embodiment described above can be obtained.

Note that, as for the number of slits 36s, any number is possible in addition to one, two and four as described above if the number is such that can cause small pieces of the outer peripheral side potion to be elastically deformed to the inner side in the diameter direction after the fitted member 37 is fitted over the outer face 36g of the outer peripheral side potion of the inserting portion 36.

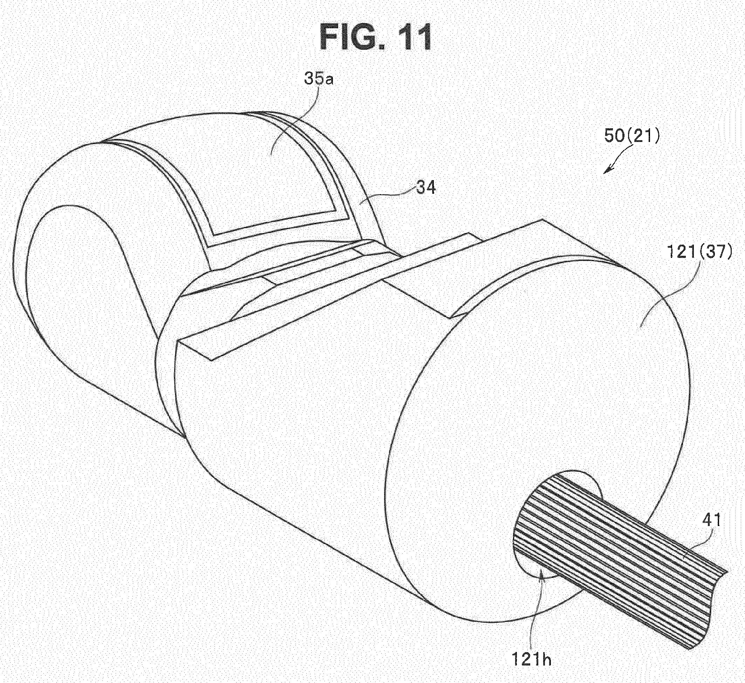

Further, another modification will be shown below with use of Fig. 11. Fig. 11 is a partial perspective view showing a modification of the distal end side of the ultrasound probe in which the fitted member of Fig. 3 is configured with a distal end rigid member.

As shown in Fig. 11, the fitted member 37 may be configured with a distal end rigid member 121 constituting the distal end portion 21 and provided with the image pickup unit, the illumination unit and the like not shown. That is, in Fig. 11, the distal end rigid member 121 also serves as the fitted member 37.

In this case, an inner peripheral face of a channel 121h which is formed so as to pass through the distal end rigid member 121 along the extension direction E is fitted on the outer peripheral side potion of the inserting portion 36 and causes the outer peripheral side potion to be elastically deformed to the inner side in the diameter direction.

According to such a configuration, the fitted member 37 may not be separately provided in order to cause the outer peripheral side potion of the inserting portion 36 to be elastically deformed to the inner side in the diameter direction as in the present embodiment described above because the fitted member 37 has the configuration of being inserted in the channel 121h of the distal end rigid member 121 in the present embodiment also though it is not shown.

Therefore, it is possible to cause the diameter of the distal end portion 21 to be smaller than that of the present embodiment described above by a thickness of the fitted member 37. Note that other effects are same as those of the present embodiment described above.

### (Second embodiment)

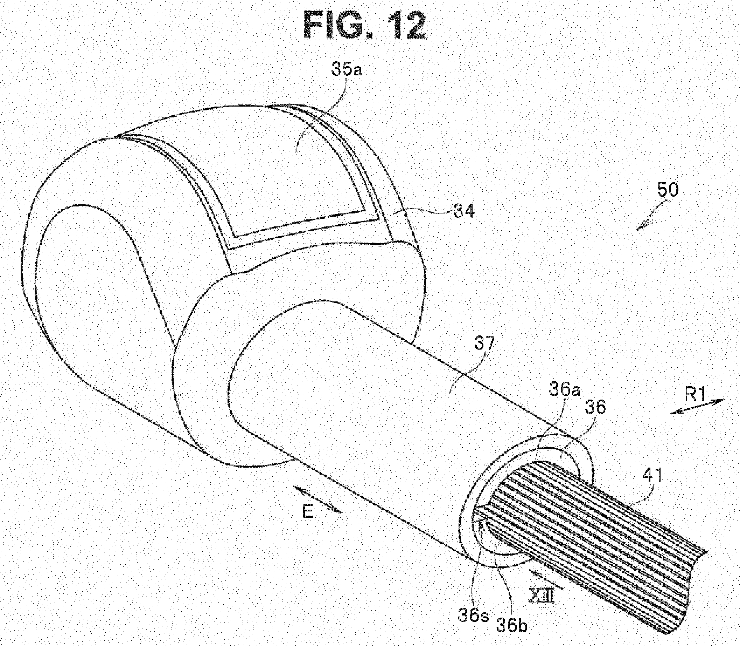

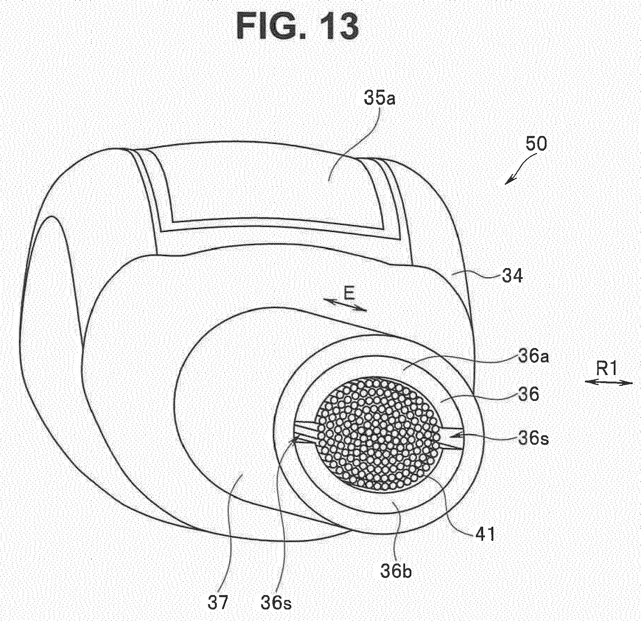

Fig. 12 is a partial perspective view showing the distal end side of the ultrasound probe of an ultrasound endoscope of the present embodiment from which the insulating tube has been removed; and Fig. 13 is a diagram of the distal end side of the ultrasound probe of Fig. 12 when seen from a XIII direction in Fig. 12.

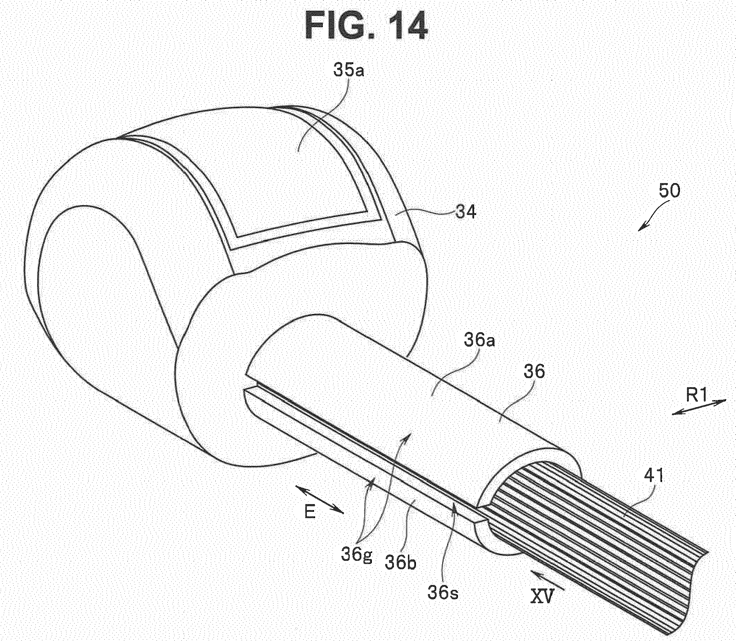

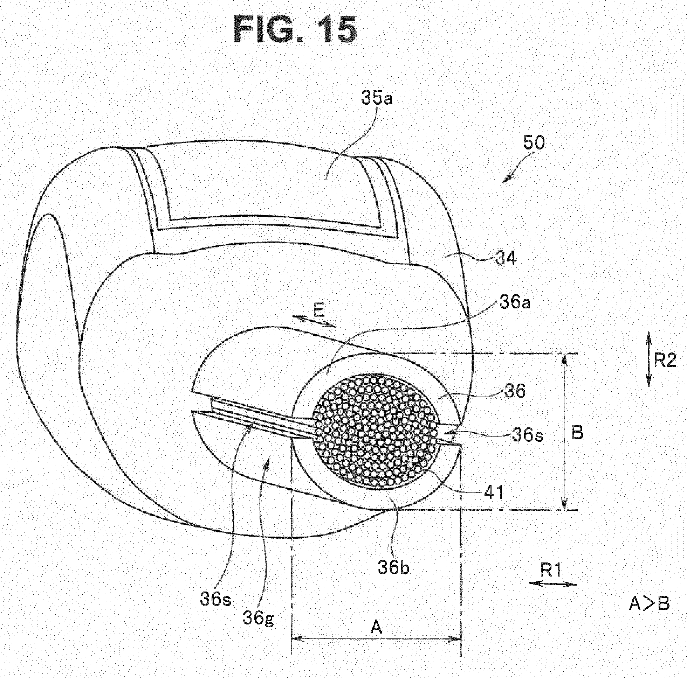

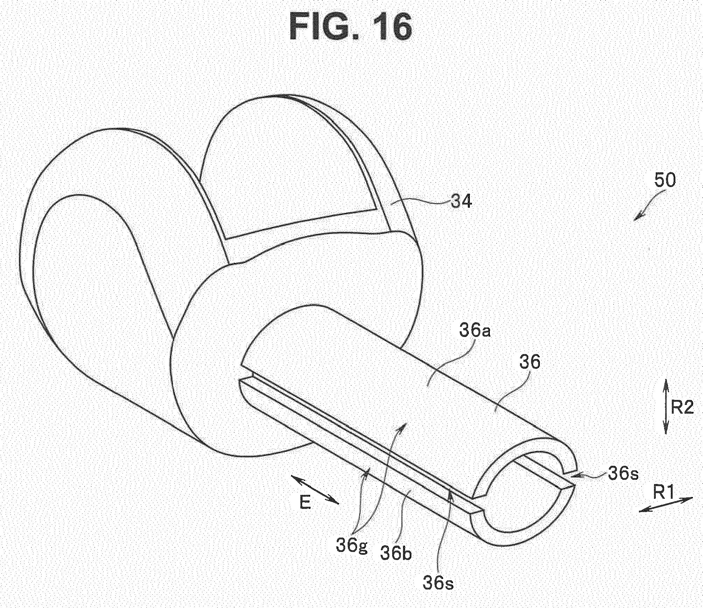

Further, Fig. 14 is a partial perspective view showing the distal end side of the ultrasound probe of Fig. 12 from which the fitted member has been removed; Fig. 15 is a diagram of the distal end side of the ultrasound probe of Fig. 14 when seen from a XV direction in Fig. 14; and Fig. 16 is a perspective view showing only the housing portion and the inserting portion extracted from the distal end side of the ultrasound probe of Fig. 12.

A configuration of the ultrasound endoscope of the second embodiment is different in a point that the inserting portion has an elliptical-cylindrical shape, in comparison with the ultrasound endoscope of the first embodiment shown in Figs. 1 to 11 described above. Therefore, components similar to those of the first embodiment will be given same reference numerals, and description of the components will be omitted.

As shown in Figs. 12 to 16, the inserting portion 36 is extended from the proximal end face of the housing portion 34 to rearward, having an elliptical-cylindrical shape in the present embodiment.

More specifically, as shown in Figs. 13 and 15, the inserting portion 36 is extended, having an elliptical-cylindrical shape elongated in the lateral direction R1, with a diameter A in the lateral direction R1 larger than a diameter B in the longitudinal direction R2 (A>B).

Note that other components are same as those of the first embodiment described above. Further, positions where the slits 36s are to be formed may be in the longitudinal direction R2 shown in Fig. 9 described above; the number of slits 36s to be formed may be any number; and, as for the fitted member 37, the distal end rigid member 121 may also serve as the fitted member 37 as shown in Fig. 11.

According to such a configuration, it is possible to secure the clearance S between the outer periphery of the cables 41 and the inner face 36n of the outer peripheral side potion of the inserting portion 36 before the fitted member 37 is fitted, largely in the lateral direction R1, and, therefore, the workability of inserting the cables 41 into the inserting portion 36 is improved. Additionally, since it is possible to cause the clearance S in the longitudinal direction R2 before the fitted member 37 is fitted to be smaller than that of the first embodiment described above, it is possible to reduce the diameter of the inserting portion 36 in the longitudinal direction R2 more than the first embodiment described above.

Note that the configuration of the present embodiment is especially effective when, at the time of providing the distal end side of the ultrasound probe 50 inside the distal end rigid member 121, an outer diameter dimension in the longitudinal direction R2 is insufficient, and outer diameter dimension in the lateral direction R1 is sufficient in the distal end rigid member 121.

Note that other effects are similar to those of the first embodiment described above.

### (Third embodiment)

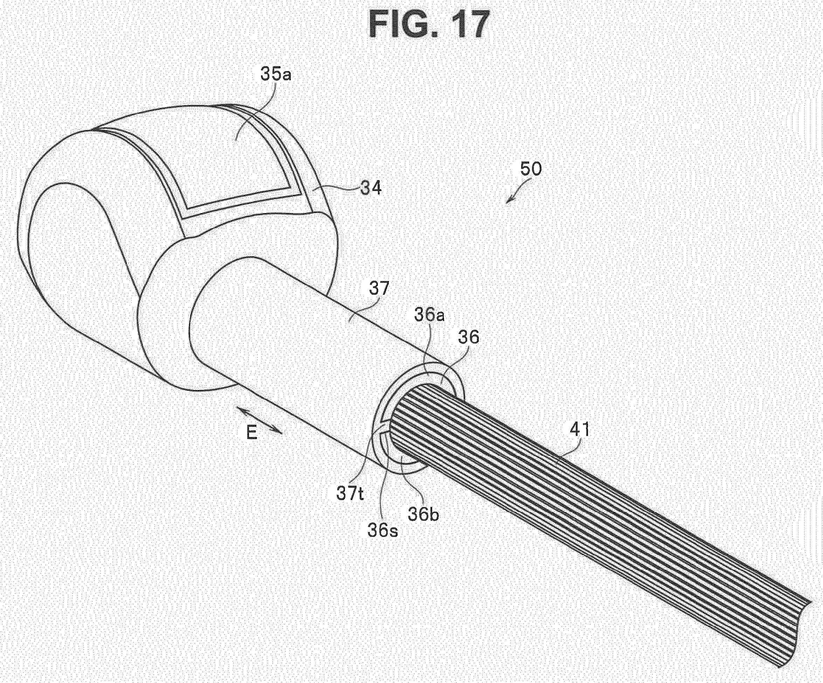

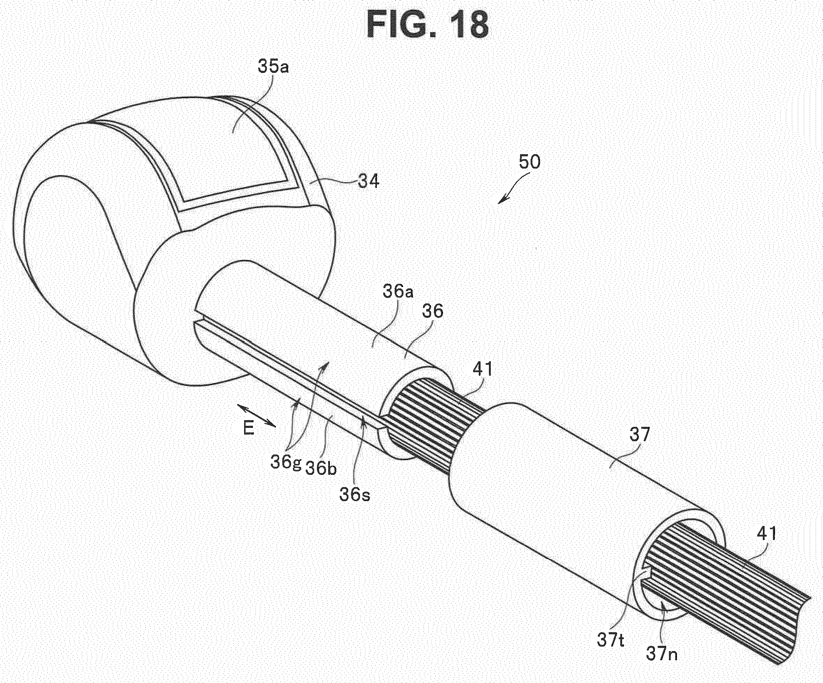

Fig. 17 is a partial perspective view showing the distal end side of the ultrasound probe of an ultrasound endoscope of the present embodiment from which the insulating tube has been removed; and Fig. 18 is a partial perspective view showing a state in which the fitted member is pulled out from the outer face of the outer peripheral side portion of the inserting portion on the distal end side of the ultrasound probe of Fig. 17.

A configuration of the ultrasound endoscope of the third embodiment is different in a point that projection portions are formed on an inner face of the fitted member, in comparison with the ultrasound endoscope of the first embodiment shown in Figs. 1 to 11 and the ultrasound endoscope of the second embodiment shown in Figs. 12 to 16, which have been described above. Therefore, components similar to those of the first and second embodiments will be given same reference numerals, and description of the components will be omitted.

As shown in Figs. 17 and 18, in the present embodiment, projection portions 37t to be fitted with the slits 36s of the inserting portion 36 are formed on an inner face 37n of the fitted member 37 with a length corresponding to a range of a slit 36s formed in the extension direction E. Further, the same number of projection portions 37t as the number of the slits 36s are formed on the inner face 37n.

Note that other components are same as those of the first embodiment described above. Further, positions where the slits 36s are to be formed may be in the longitudinal direction R2 shown in Fig. 9 described above, and the number of slits 36s to be formed may be any number.

Furthermore, as for the fitted member 37, the distal end rigid member 121 may also serve as the fitted member 37 as shown in Fig. 11 described above, and the inserting portion 36 may be extended, having an elliptical-cylindrical shape as in the second embodiment described above.

According to such a configuration, even if the thickness T of the outer peripheral side potion of the inserting portion 36 is such that cannot ensure the prescribed voltage resistance of the cables 41, the projection portions 37t fit with the slits 36s and block the slits 36s when the fitted member 37 is fitted over the outer face 36g of the outer peripheral side potion of the inserting portion 36.

According to the above, since the fitted member 37 can also function as a member for ensuring the prescribed voltage resistance of the cables 41 together with the inserting portion 36, it is only required that the thickness T of the outer peripheral side potion of the inserting portion 36 + the thickness of the fitted member 37 is the minimum thickness to ensure the electrical safety standards of the cables 41. Therefore, even if the thickness T of the outer peripheral side potion of the inserting portion 36 is thin, the electrical safety standards of the cables 41 can be sufficiently ensured.

Note that other effects are similar to those of the first and second embodiments described above.

### (Fourth embodiment)

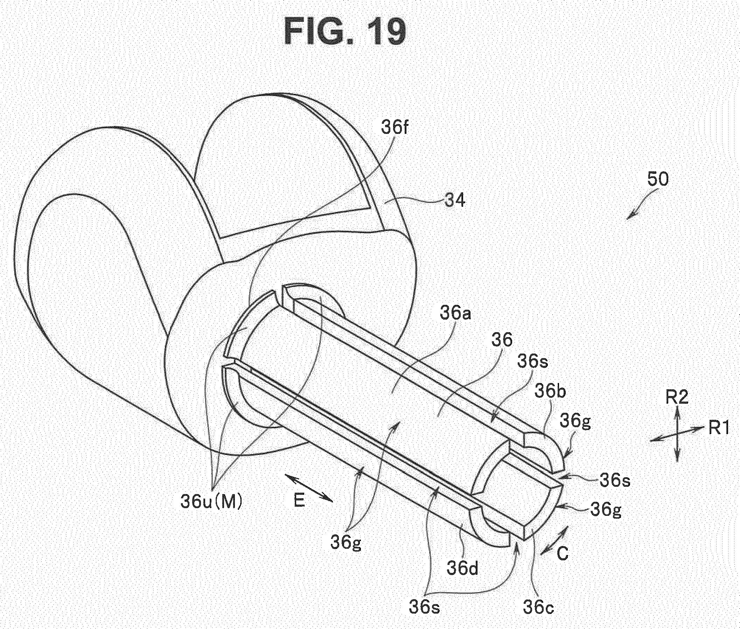

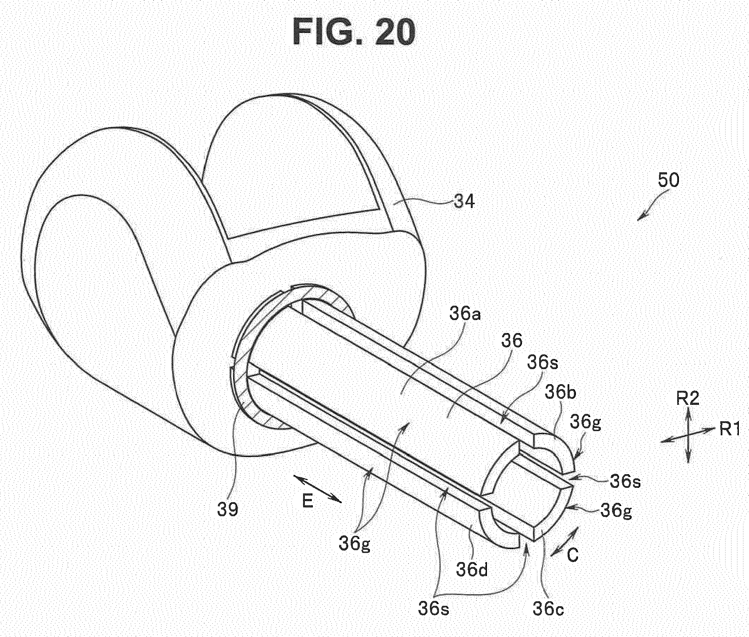

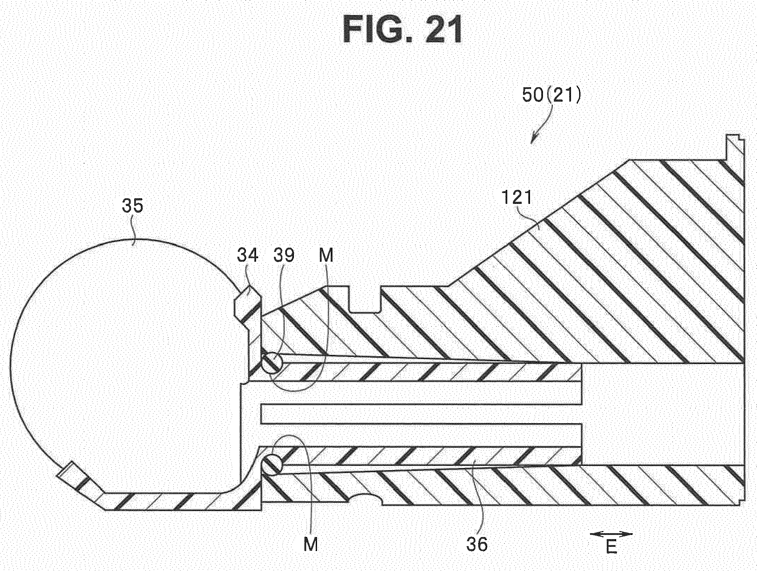

Fig. 19 is a partial perspective view showing the distal end side of the ultrasound probe of an ultrasound endoscope of the present embodiment by extracting only the housing portion and the inserting portion; Fig. 20 is a partial perspective view showing a state in which a watertight member is fitted in a groove of the inserting portion of Fig. 19; and Fig. 21 is a partial cross-sectional view of the distal end side of the ultrasound probe when the distal end rigid member is fitted on the outer face of the outer peripheral side portion of the inserting portion of Fig. 20.

A configuration of the ultrasound endoscope of the fourth embodiment is different in a point that a thin-thickness portion is formed at a part of each small piece of the inserting portion, which is in contact with the housing portion, in comparison with the ultrasound endoscope of the first embodiment shown in Figs. 1 to 11, the ultrasound endoscope of the second embodiment shown in Figs. 12 to 16, and the ultrasound endoscope of the third embodiment shown in Figs. 17 and 18, which have been described above. Therefore, components similar to those of the first to third embodiments will be given same reference numerals, and description of the components will be omitted.

As shown in Fig. 19, thin-thickness portions 36u, which are thinner than other parts of the outer peripheral side potion of the inserting portion 36, are formed at parts 36f of the four small pieces 36a to 36d of the outer peripheral side potion of the inserting portion 36, which are in contact with the housing portion 34, respectively.

The respective thin-thickness portions 36u are such that reduce stress concentrated on the respective parts 36f of the respective small pieces 36a to 36d which are elastically deformed, and each thin-thickness portion 36u has a groove M which is circumferentially formed in the circumferential direction C.

A watertight member 39 such as an O-shaped ring is fitted in the grooves M as shown in Figs. 20 and 21. As shown in Fig. 21, when the fitted member 37 also serves as the distal end rigid member 121, the watertight member 39 secures watertightness between the inserting portion 36 and the distal end rigid member 121.

Note that other components are same as those of the first embodiment described above. Further, the number of slits 36s to be formed may be any number; the inserting portion 36 may be extended, having an elliptical-cylindrical shape as in the second embodiment described above; and the projection portions to be fitted with the slits 36s may be provided on the distal end rigid member 121.

According to such a configuration, it is possible to reduce stress added to the respective parts 36f accompanying elastic deformation of the respective small pieces 36a to 36d by the thin-thickness portions 36u when the distal end rigid member 121 is fitted on the outer face 36g of the outer peripheral side potion of the inserting portion 36. Additionally, it is possible to secure watertightness between the inserting portion 36 and the distal end rigid member 121 by the watertight member 39 fitted with the grooves M of the thin-thickness portions 36u. That is, the thin-thickness portions 36u serve as such that perform the function of reducing stress of the respective small pieces 36a to 36d and serve as a position where the watertight member 39 is to be arranged.

Note that other effects are same as those of the first to third embodiments described above.

### (Fifth embodiment)

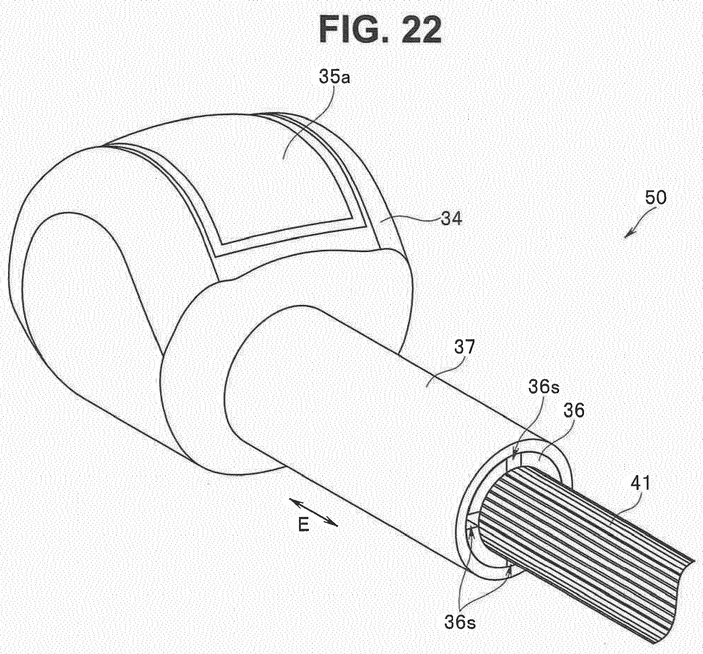

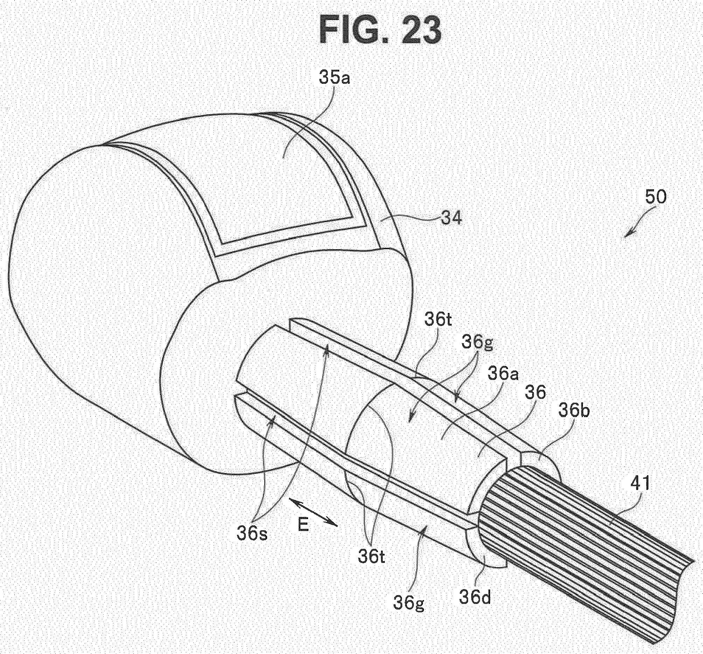

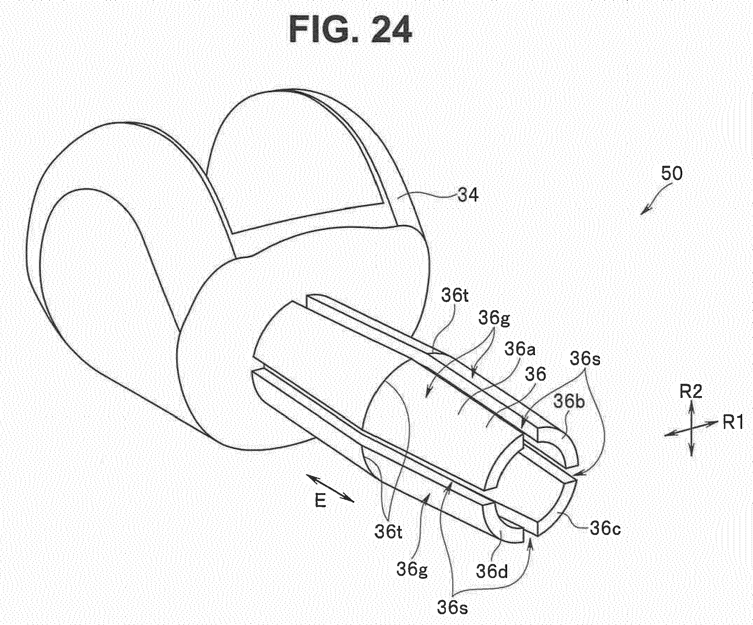

Fig. 22 is a partial perspective view showing the distal end side of the ultrasound probe of an ultrasound endoscope of the present embodiment from which the insulating tube has been removed; Fig. 23 is a partial perspective view showing the distal end side of the ultrasound probe of Fig. 22 from which the fitted member has been removed; and Fig. 24 is a partial perspective view showing only the housing portion and the inserting portion extracted from the distal end side of the ultrasound probe of Fig. 22.

A configuration of the ultrasound endoscope of the fifth embodiment is different in a point that retaining projections for the fitted portion are provided on the outer face of the outer peripheral side potion of the inserting portion, in comparison with the ultrasound endoscope of the first embodiment shown in Figs. 1 to 11, the ultrasound endoscope of the second embodiment shown in Figs. 12 to 16, the ultrasound endoscope of the third embodiment shown in Figs. 17 and 18, and the ultrasound endoscope of the fourth embodiment shown in Figs. 19 to 21, which have been described above. Therefore, components similar to those of the first to fourth embodiments will be given same reference numerals, and description of the components will be omitted.

As shown in Figs. 23 and 24, retaining projections 36t for the fitted member 37 swelling to the outer side in the diameter direction may be provided on substantially middle parts of the outer faces 36g of the respective small pieces 36a to 36d of the outer peripheral side potion of the inserting portion 36 in the extension direction E, respectively.

Note that other components are same as those of the first embodiment described above. Further, the number of slits 36s to be formed may be any number; and, furthermore, the distal end rigid member 121 may also serve as the fitted member 37 as shown in Fig. 11.

Further, the inserting portion 36 may be extended, having an elliptical-cylindrical shape as in the second embodiment described above; the projection portions 37t may be formed on the inner face 37n of the fitted member 37 as in the third embodiment described above; and the thin-thickness portions 36u may be formed on the respective parts 36f of the respective small piece portions 36a to 36d, respectively, as in the fourth embodiment described above.

According to such a configuration, when the fitted member 37 is fitted on the outer face 36g of the outer peripheral side potion of the inserting portion 36 as shown in Fig. 22, the fitted member 37 is prevented from coming off rearward by the retaining projections 36t.

Note that other effects are same as those of the first to fourth embodiments described above.

By the way, as shown in Fig. 2 described above, the plurality of connectors 45 are provided on the proximal end side of the cables 41. Note that each connector 45 is configured, for example, with a flexible substrate in a thin plate shape.

However, since an outer diameter of each connector 45 is larger than that of the plurality of cables 41 as shown in Fig. 2, there is a problem that it is difficult for the connectors 45 to pass through the inserting portion 36.

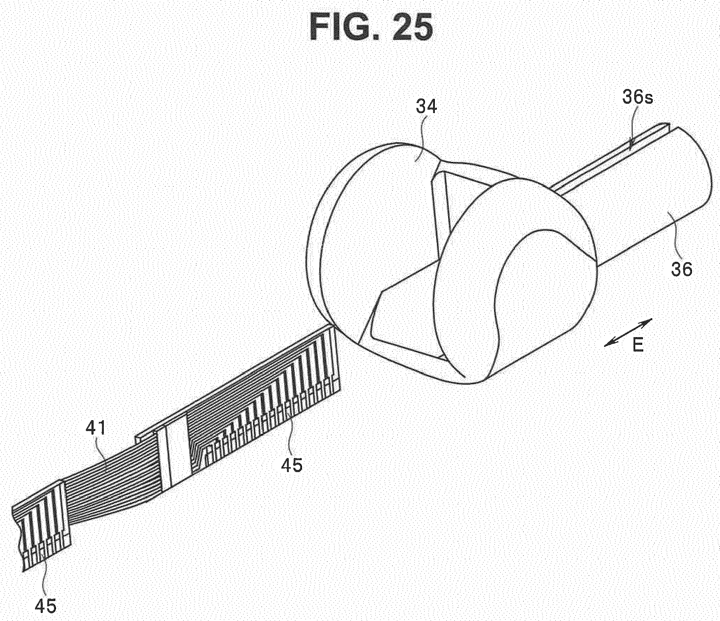

A procedure for causing the cables 41 and the connectors 45 to pass through the inserting portion 36 of Fig. 9 will be shown below with use of Figs. 25 to 27.

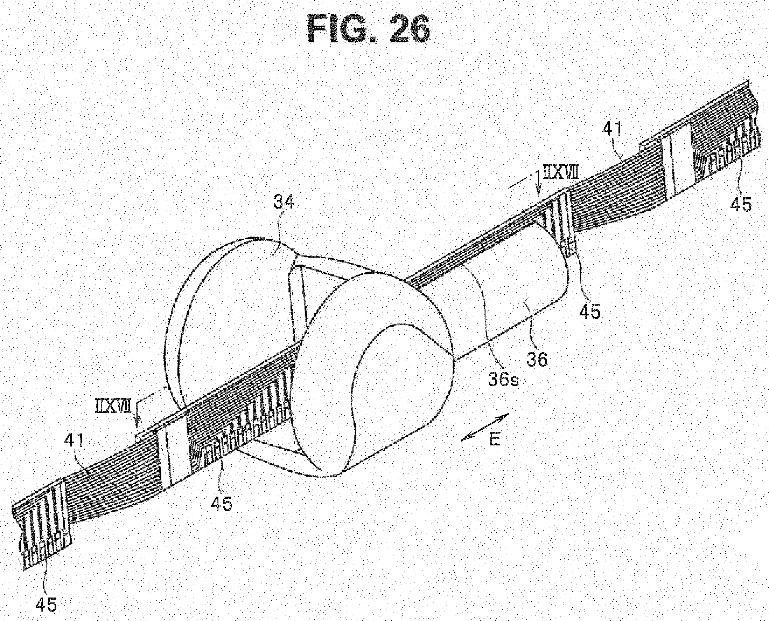

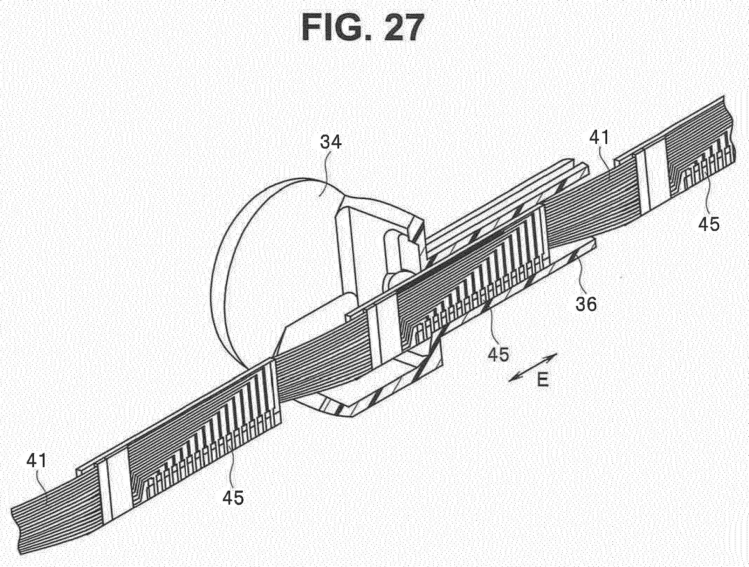

Fig. 25 is a partial perspective view showing a state in which the cables and the connectors are caused to be close to the housing portion and the inserting portion of Fig. 9 from frontward; Fig. 26 is a partial perspective view showing a state in which the cables and the connectors of Fig. 25 are caused to be inserted into the housing portion and the inserting portion of Fig. 25; and Fig. 27 is a diagram showing sections of the housing portion and the inserting portion along a IIXVII-IXVII line in Fig. 26 together with the cables and the connectors.

In a case of causing the cables 41 and the connectors 45 to pass through the housing portion 34 and the inserting portion 36, the worker causes the proximal end side of the cables 41 and the connectors 45 to be close to the housing portion 34 and the inserting portion 36 from frontward of the housing portion 34 as shown in Fig. 25 first.

After that, as shown in Figs. 26 and 27, the worker causes the cables 41 and the connectors 45 to pass through the housing portion 34 and the inserting portion 36.

At this time, by causing the connectors 45 to pass through along the slits 36s formed on the inserting portion 36, it is possible to cause the connectors 45 to pass through with good workability using the slits 36s, without necessity of adjusting the inner diameter of the inserting portion 36 to the outer diameter of the connectors 45. Therefore, it is possible to improve passage of the connectors 45 through the inserting portion 36 and realize reduction in the diameter of the inserting portion 36.

The present application is filed, claiming priority based on Japanese Patent Application No. 2014-243427 filed to Japan on December 1, 2014, the content of which is incorporated in the specification, Claims and drawings of this application by reference thereto.

## Claims

1. An ultrasound endoscope comprising:
an insertion portion configured to be inserted into an observation site and provided with a housing portion on a distal end side in an extension direction;
an ultrasound transmitting/receiving portion provided in the housing portion and configured to transmit and receive ultrasound to and from the observation site;
a cable extended rearward in the extension direction from the ultrasound transmitting/receiving portion and configured to transmit electric signals to be inputted to and outputted from the ultrasound transmitting/receiving portion;
an inserting portion extended rearward in the extension direction from the housing portion, having a substantially cylindrical shape, with a slit along the extension direction being formed on an outer peripheral side potion, the inserting portion being configured to cause the cable to extend from an inside of the housing portion; and
a fitted member fitted on an outer face of the outer peripheral side potion of the inserting portion so as to cover at least a part of the outer face along the extension direction.

2. The ultrasound endoscope according to claim 1, wherein the inserting portion is extended from the housing portion, having an elliptical-cylindrical shape.

3. The ultrasound endoscope according to claim 1 or 2, wherein a projection portion configured to be fitted in the slit of the inserting portion is provided on an inner face of the fitted member.

4. The ultrasound endoscope according to any one of claims 1 to 3, wherein, on the outer peripheral side potion of the inserting portion, a thin-thickness portion thinner than other parts of the inserting portion is formed on at least a part of a portion in contact with the housing portion in a circumferential direction.

5. The ultrasound endoscope according to claim 4, wherein
the thin-thickness portion includes a groove circumferentially formed on the outer face of the outer peripheral side potion of the inserting portion; and
a watertight member against the fitted member is fitted in the groove.

6. The ultrasound endoscope according to any one of claims 1 to 5, wherein a retaining projection for the fitted member is provided on the outer face of the outer peripheral side potion of the inserting portion.

7. The ultrasound endoscope according to any one of claims 1 to 6, wherein the fitted member is a distal end rigid member constituting a distal end portion positioned on a distal end side of the insertion portion in the extension direction.
